# EUROPEAN PATENT APPLICATION

(11) **EP 4 610 348 A1**
(43) Date of publication of application: **03.09.2025**
(21) Application number: 23869790.8
(22) Date of filing: 27.06.2023
(51) Int. Cl.: C12N 5/10, C12N 5/071, A61K 35/407, A61P 43/00

(54) **HEPATOLENTICULAR DEGENERATION CELL, PREPARATION METHOD THEREFOR, AND EVALUATION METHOD THEREFOR**

(30) Priority: 27.09.2022 CN 202211181622
(71) Applicant: GUANGXIU-GAOXIN LIFE SCIENCES CO., LTD. HUNAN, Changsha, Hunan 410205 (CN)
(72) Inventor: SUN, Yi, Changsha, Hunan 410205 (CN); OUYANG, Qi, Changsha, Hunan 410205 (CN); LIN, Ge, Changsha, Hunan 410205 (CN); LU, Guangxiu, Changsha, Hunan 410205 (CN)
(74) Representative: Herrero & Asociados, S.L.
(86) International application number: PCT/CN2023/102807
(87) International publication number: WO 2024/066542

(57) **Abstract**

Provided are a hepatolenticular degeneration cell, a preparation method therefor, and an evaluation method therefor. The hepatolenticular degeneration cell is prepared by performing in-vitro directional induced differentiation on a human embryonic stem cell line carrying an ATP7B gene mutation. According to the preparation method for the hepatolenticular degeneration cell, a human embryonic stem cell carrying an ATP7B gene variation is used to be subjected to in-vitro induction into a hepatocyte, such that a hepatocyte with a hepatolenticular degeneration disease phenotype is obtained, which can reflect the influence of a copper ion on a human liver cell, and the influence is highly related to the physiological and pathological conditions of a human body, thereby providing a new way for pathogenesis, new drug research and development and drug hepatotoxicity research of a hepatolenticular degeneration disease.

## Description

### TECHNICAL FIELD

The present application relates to the field of biotechnology, in particular to hepatolenticular degeneration cells, a preparation method thereof, and an evaluation method thereof.

### BACKGROUND

Hepatolenticular degeneration is a rare autosomal recessive genetic disease characterized by primary copper metabolism disorder. Hepatolenticular degeneration is caused by ATP7B gene mutations. More than 800 types of ATP7B gene mutations have been reported. The ATP7B gene mutations lead to copper ion transport and metabolism disorders in the body. Excessive copper ion accumulation in hepatocytes causes necrosis of hepatocytes. Accumulation of copper ions in various organs in the body leads to damage to the various organs throughout the body and a series of clinical manifestations, such as liver abnormalities including acute and chronic liver damage, liver cirrhosis, chronic hepatitis and eventually liver failure, neurological defects, extrapyramidal signs and mental symptoms, corneal K-F pigment rings, acute hemolysis, bone and joint pain, etc.

Drug therapy is currently an important means for clinically alleviating the symptoms of hepatolenticular degeneration. Conventional research on hepatolenticular degeneration is mainly based on animal models, such as LEC rats, TX mice, and Atp7b -/mice. The above animal models have played a vital role in the study of the mechanism of hepatolenticular degeneration, target validation, and new drug development. Due to species differences, humans show fundamental differences in physiological functions, metabolism, and genetics compared to model animals. Therefore, animal models neither can fully simulate the clinical manifestations of human patients with hepatolenticular degeneration, nor can they accurately predict adverse drug reactions when patients suffering from the hepatolenticular degeneration take drugs. The animal models are also limited by long modeling cycles, high costs, susceptibility to interference in detection indicators, and ethical concerns. Therefore, new drug research and development for conventional hepatolenticular degeneration has very low efficiency.

The difficulty in improving the efficiency of new drug research and development for hepatolenticular degeneration lies in how to develop a disease model with high clinical predictability, low costs, and a short modeling cycle.

### SUMMARY

In order to solve the above problem, a first objective of the present application is to provide a method for preparing a hepatolenticular degeneration cell, including *in vitro* directedly inducing a human embryonic stem cell line carrying an ATP7B gene mutation to differentiate into a hepatocyte, thereby forming the hepatolenticular degeneration cell. As such, the hepatolenticular degeneration model exhibits improved clinical predictability, reduced modeling costs, and shortened modeling cycles, thereby improving the efficiency of new drug research and development for hepatolenticular degeneration.

In an embodiment of the present application, the ATP7B gene mutation satisfies at least one of the following characteristics:
(1) the ATP7B gene mutation includes the c.1881_1882insT homozygous mutation;
(2) the ATP7B gene mutation includes the c.2333G>T homozygous mutation; and
(3) the ATP7B gene mutations include c.2333G > T and c.1881_1882insT compound heterozygous mutations.

In an embodiment of the present application, the method further includes:
*in vitro* directedly inducing the human embryonic stem cell line carrying the ATP7B gene mutation to sequentially go through a definitive endoderm induction stage, a liver lineage-directed differentiation stage, and a hepatocyte maturation promotion stage to differentiate into a hepatocyte, thereby forming the hepatolenticular degeneration cell.

In an embodiment of the present application, a first induction medium and a second induction medium are sequentially used for induction culture in the definitive endoderm induction stage, wherein:
the first induction medium is a RPMI 1640 medium supplemented with activin A and Wnt3a; and
the second induction medium is a RPMI 1640 medium supplemented with activin A and fetal bovine serum.

In an embodiment of the present application, a concentration of activin A in the first induction medium is in a range from 5 ng/mL to 300 ng/mL;
a concentration of Wnt3a in the first induction medium is in a range from 20 ng/mL to 100 ng/mL;
a concentration of activin A in the second induction medium is in a range from 10 ng/mL to 200 ng/mL;
a volume concentration of fetal bovine serum in the second induction medium is in a range from 0.1% to 4%.

In an embodiment of the present application, a third induction medium and a fourth induction medium are sequentially used for induction culture in the liver lineage-directed differentiation stage, wherein:
the third induction medium is a KO/DMEM medium supplemented with keratinocyte growth factor and fetal bovine serum; and
the fourth induction medium is a KO/DMEM medium supplemented with a serum substitute, L-glutamine, a non-essential amino acid, beta-mercaptoethanol, and dimethyl sulfoxide.

In an embodiment of the present application, a concentration of the keratinocyte growth factor in the third induction medium is in a range from 15 ng/mL to 100 ng/mL;
a volume concentration of the fetal bovine serum in the third induction medium is in a range from 1% to 3%;
a volume concentration of the serum substitute in the third induction medium is 20%;
a concentration of L-glutamine in the third induction medium is in a range from 1 mM to 3 mM;
a volume concentration of the non-essential amino acid in the third induction medium is in a range from 0.1% to 15%;
a concentration of beta-mercaptoethanol in the third induction medium is in a range from 0.1 mM to 0.12 mM; and
a volume concentration of dimethyl sulfoxide in the third induction medium is in a range from 0.2% to 2%.

In an embodiment of the present application, a fifth induction medium is used for induction culture in the hepatocyte maturation promotion stage, and the fifth induction medium is an L-15 medium supplemented with hepatocyte growth factor, oncostatin, dexamethasone, L-glutamine, and fetal bovine serum.

In an embodiment of the present application, a concentration of the hepatocyte growth factor in the fifth induction medium is in a range from 10 ng/mL to 300 ng/mL;
a concentration of oncostatin in the fifth induction medium is in a range from 1 ng/mL to 150 ng/mL;
a concentration of dexamethasone in the fifth induction medium is in a range from 0.05 µM to 1.2 µM;
a concentration of L-glutamine in the fifth induction medium is in a range from 2 mM to 3 mM; and
a volume concentration of fetal bovine serum in the fifth induction medium is 10%.

In an embodiment of the present application, the method for preparing the human embryonic stem cell line carrying the ATP7B gene mutation includes the steps of:
culturing an embryo carrying the ATP7B gene mutation in a blastocyst culture medium, mechanically excising an inner cell mass, and culturing the inner cell mass on mouse embryonic fibroblast feeder cells irradiated with gamma rays; and
picking and stably culturing clonal undifferentiated growing cells to generate spreading clones, thereby preparing the embryonic stem cell line for hepatolenticular degeneration.

A second objective of the present application is to provide a hepatolenticular degeneration cell, which is prepared by the preparation method described above.

A third objective of the present application is to provide a method for evaluating a hepatolenticular degeneration cell, including incubating the hepatolenticular degeneration cell described above in a culture medium containing copper ions, and evaluating effects of copper ions on the hepatolenticular degeneration cell before and after incubation.

In an embodiment of the present application, evaluating the effects of copper ions on the hepatolenticular degeneration cell before and after incubation specifically includes:
detecting at least one of the following in the hepatolenticular degeneration cell before and after incubation: expression levels of ATP7B gene and ATP7B protein, cell vitality, copper ion metabolism capacity, and an oxidative stress response.

In an embodiment of the present application, when at least one of the expression levels of ATP7B gene and ATP7B protein, the copper ion metabolism capacity, and the oxidative stress response in the hepatolenticular degeneration cell before and after incubation is detected, a concentration of copper ions is in a range from 100 µM to 300 µM;
detecting the cell vitality of the hepatolenticular degeneration cell before and after incubation specifically includes: detecting the cell viability of the hepatolenticular degeneration cell in different culture media containing different concentrations of copper ions before and after incubation, wherein the concentrations of copper ions in the different culture media are in a range from 0 mM to 1.5 mM.

In an embodiment of the present application, the incubation lasts for 24 to 48 hours, and the culture medium containing copper ions is replaced every 22 to 26 hours.

In an embodiment of the present application, the copper ions are derived from copper sulfate or copper chloride.

In an embodiment of the present application, the evaluation method further includes determining the sublocalization of the ATP7B protein in the hepatolenticular degeneration cell prepared by the preparation method described above.

A fourth objective of the present application is to provide use of the hepatolenticular degeneration cell in the manufacture of a medicament for treating a hepatolenticular degeneration disease.

The preparation method of hepatolenticular degeneration cells in the present application creatively *in vitro* induces the human embryonic stem cell carrying the ATP7B gene mutation to differentiate into a hepatocyte and obtains the hepatocyte with hepatolenticular degeneration disease phenotypes, which can truly reflect the influence of copper ions on human hepatocytes that is highly correlated with the physiological and pathological conditions of the human body, thereby providing a new disease model for pathogenic mechanism research, new drug development, or drug hepatotoxicity research for a hepatolenticular degeneration disease, and providing the advantages of high clinical predictability, short modeling cycle, and low costs.

### BRIEF DESCRIPTION OF THE DRAWINGS

FIG. 1 shows schematic images during the establishment of a human embryonic stem cell line carrying an ATP7B gene mutation in Example 1 of the present application. FIG. 1A shows a blastocyst of an embryo carrying the ATP7B gene mutation on the sixth day of *in vitro* incubation. FIG. 1B shows small clones that appear after 3 to 5 days of *in vitro* culture. FIG. 1C shows *ch*HES-391 cells obtained after stably culturing on MEF.
FIG. 2 shows a sequencing map of the ATP7B gene in *ch*HES-391 cells in Example 1 of the present application.
FIG. 3 shows schematic images of morphologies of *ch*HES-391 cells at various induction stages in Example 2 of the present application. There are *ch*HES-391 cells, cells in a first stage of induced differentiation of *ch*HES-391 cells, cells in a second stage of induced differentiation of *ch*HES-391 cells, and cells in a third stage of induced differentiation of *ch*HES-391 cells from left to right.
FIG. 4 is a graph showing flow cytometry results of hepatolenticular degeneration cells in Example 2 of the present application.
FIG. 5 shows experimental results of PAS glycogen staining and Oil Red-O staining of hepatolenticular degeneration cells in Example 2 of the present application.
FIG. 6 is a schematic graph showing detection results of ATP7B gene expression levels of *ch*HES-391-derived hepatocytes before and after incubation with copper ions in Example 3 of the present application.
FIG. 7 is a schematic graph showing detection results of ATP7B protein expression levels of *ch*HES-391-derived hepatocytes before and after incubation with copper ions in Example 3 of the present application.
FIG. 8 is a schematic graph showing detection results of subcellular localization of ATP7B protein in *ch*HES-391-derived hepatocytes in Example 3 of the present application.
FIG. 9 shows detection results of effects of copper ion accumulation on cell viability of *ch*HES-391-derived hepatocytes in Example 3 of the present application.
FIG. 10 shows schematic detection results of copper ion metabolism ability of *ch*HES-391-derived hepatocytes in Example 3 of the present application.
FIG. 11 shows schematic detection results of copper ion accumulation on the oxidative stress response of *ch*HES-391-derived hepatocytes in Example 3 of the present application.

### DETAILED DESCRIPTION

References to embodiments of the present application are now provided in detail, one or more examples of which are described below. Each example is provided as an explanation rather than a limitation of the present application. In fact, it is apparent to those of ordinary skill in the art that various modifications and variations can be made without departing from the scope or spirit of the present application. For example, features illustrated or described as a part of one embodiment may be used in another embodiment to form a further embodiment.

Therefore, the above modifications and variations are intended to be covered by the present application as falling within the scope of the appended claims and their equivalents. Other objects, features and aspects of the present application are disclosed in or are apparent from the following detailed description. Those of ordinary skill in the art appreciate that this discussion is merely a description of exemplary embodiments and is not intended to limit the broader aspects of the present application.

As mentioned above, when used in the development of drugs for hepatolenticular degeneration, conventional animal models neither can fully simulate the clinical manifestations of patients with hepatolenticular degeneration, nor accurately predict some adverse drug reactions generated when patients take drugs. Conventional animal models are also limited by long modeling cycles, high costs, susceptibility to interference in detection indicators, and ethical concerns.

*In vitro* culturing cells can allow for direct and dynamic observation and study of the morphology, structure, and function of hepatocytes, with advantages of repeatability and abundant sources, which are of great significance in the study of physiology, pathology, and pharmacology of liver. At the cellular level, human primary hepatocyte is often used as the cell for studying liver diseases such as hepatolenticular degeneration, and is used as a gold standard for the study of disease mechanisms, drug treatment regimens, and adverse drug reactions. However, due to defects such as scarce sources, large heterogeneity between different batches, unstable phenotypes, short lifespan, and easy dedifferentiation in *in vitro* culture, its application in practical research is limited.

Although cells derived from human liver cancer cell lines can be obtained in large quantities and are easy to manipulate, the hepatocytes derived from liver cancer cell lines show defective signaling pathways, which results in unapparent disease manifestations, unstable inheritance, and lack of specific drug-metabolizing enzymes, thereby greatly limiting their applicability.

With the development of stem cell technology, establishing liver disease models by human stem cells has provided a new approach for the study of related pathogenic mechanisms and the screening of new drugs. Human pluripotent stem cells are a general term for cells with unlimited self-renewal and multidirectional differentiation potential. Human Embryonic Stem Cells (hESCs) and Human Induced Pluripotent Stem Cells (hiPSCs) are main types for research.

*In vitro* studies of hepatolenticular degeneration are currently mainly based on cells derived from liver cancer cell lines and induced pluripotent stem cells. Hepatocytes differentiated from induced pluripotent stem cells derived from skin fibroblasts of patients can reproduce the main disease phenotypes of hepatolenticular degeneration, but as a disease model, still have the following problems. (1) Although the induction method of hiPSCs has been continuously improved, the production efficiency of hiPSCs is still lower than that of hESCs, and many hiPSCs cannot support effective normal differentiation. (2) The genome or epigenome of hiPSCs may gradually change in the culture process.

In order to solve at least one of the above problems, a first aspect of the present application provides a method for preparing a hepatolenticular degeneration cell, including *in vitro* directedly inducing a human embryonic stem cell line carrying an ATP7B gene mutation to differentiate into a hepatocyte, thereby forming the hepatolenticular degeneration cell.

Specifically, hepatolenticular degeneration is a disease caused by mutations in the ATP7B gene (OMTM*606882) located on chromosome 13q14.3. The ATP7B gene belongs to a member of P1B-type ATPase family, which contains 21 exons and 20 introns, transcribes 6655 bp of mRNA (NM_000053.3), encodes 1465 amino acids (NP_00004), and produces a multi-transmembrane P-type copper transport ATPase (i.e., ATP7B protein). The ATPase includes 6 N-terminal metal binding domains (MBDs, each of MBDs contains a highly conserved repeating sequence of methionine-X-cysteine-X-X-cysteine), 8 transmembrane functional regions (i.e., TM-regions), 1 transduction region (i.e., N-region), 1 phosphorylation region (i.e., P-region), and 1 phosphatase region (i.e., A-region). Previous studies have showed that the function of the ATP7B protein can be completely lost if the mutated residue of the ATP7B gene is a key site for binding to ATP or copper, and partial transport function of the ATP7B protein can be retained if the mutation only affects the affinity for the substrate, slows down conformational transitions, or affects the localization of the protein.

Currently, there are more than 800 gene mutations in the ATP7B gene, which are distributed throughout the ATP7B gene. The gene mutations include types of insertion mutations, deletion mutations, frameshift mutations, splicing mutations, etc., especially missense mutations among site mutations. Most mutations occur in exons, and a few mutations occur in flanking regions of exon-intron boundaries. It has been reported that there are 18 missense mutations in the MBD region associated with exons 1 to 6, such as G85V located in MBD1, which inhibits the interaction between ATP7B and Atox-1, and affects the binding of the protein to copper. It also has been reported that there are 103 mutations in the cysteine-proline-cysteine transmembrane transport region associated with exons 7 to 10, 12, and 13, such as R778L and G937S, and mutations in this region mainly affect the function of the protein by changing its localization. There are 22 mutations in the A-region associated with exon 11, and mutations in this region can lead to hyperphosphorylation of the ATP7B protein, causing loss of catalytic activity. It is further reported that there are 47 mutations in the P-region associated with exons 14 to 16, such as I1148T. It is yet reported that there are more than 55 mutations in the N-region associated with exons 17 to 18, and mutations in this region together with the P-region affect the binding of the protein to ATP, resulting in loss of function. It can be understood that even if the amino acids replaced by mutations are present in the same region, the functions of the encoded proteins may change differently.

The ATP7B gene mutation carried by the human embryonic stem cell line of the present application includes a mutation in exon 5 and/or a mutation in exon 8. In some embodiments, the mutation includes a c.1881_1882insT homozygous mutation, which is a new pathogenic mutation. In some embodiments, the mutation further includes a c.2333G>T homozygous mutation, which is the most common pathogenic mutation in Asians. In some embodiments, the mutations include c.1881_1882insT and c.2333G > T compound heterozygous mutations. In the present application, through *in vitro* directedly inducing a human embryonic stem cell line carrying an ATP7B gene mutation to differentiate into hepatocytes, it is unexpectedly discovered that the transport function of the ATP7B protein is affected by mutations in exon 5 and mutations in exon 8 through the MBD region combined with the cysteine-proline-cysteine transmembrane transport region, which causes changes in the phenotype of hepatocytes induced by human embryonic stem cells, and shows typical pathological characteristics of hepatolenticular degeneration, such as ATP7B protein functional defects, copper ion metabolism disorders, adverse effects of copper accumulation on hepatocytes, etc., and thus can truly reflect the influence of copper ions on human hepatocytes which is highly correlated with the physiological and pathological conditions of the human body. Specifically, the mutation in exon 5 is an insertion mutation c.1881_1882insT, and the mutation in exon 8 is a missense mutation c.2333G>T (i.e., p.R778L). Among them, the insertion mutation c.1881_1882insT in exon 5 is a new mutation firstly disclosed by the present application, which indicates that the base T is inserted between positions 1881 and 1882 of the cDNA sequence of the ATP7B gene, and causes the change of the protein reading frame, resulting in a series of codon changes downstream of the insertion site, so that the gene originally encoding the ATP7B protein is changed to encode another completely different peptide chain sequence, and thus the mutation is referred to as a frameshift mutation. The missense mutation c.2333G>T in exon 8 indicates that the base G at position 2333 of the cDNA sequence of the ATP7B gene mutates to the base T, which mutates the arginine at position 778 of the ATP7B protein to lysine.

It can be understood that the embryonic stem cells carrying the ATP7B gene mutation in the present application can be obtained by isolating discarded human embryos with naturally occurring ATP7B gene mutations, or can be obtained by artificially modifying the ATP7B gene of normal human embryonic stem cells through gene editing technology.

In addition, according to the method for preparing the hepatolenticular degeneration cell provided in the present application, by creatively using the human embryonic stem cell line carrying the ATP7B gene mutation, and culturing cells in a feeder-free culture system with only added induction culture medium for the corresponding stage, hepatolenticular degeneration hepatocytes with multiple disease phenotypes can be obtained. After testing, the hepatolenticular degeneration hepatocytes exhibit high purity. The method of directional induction and differentiation into hepatocytes has a short induction cycle, high efficiency, obtains hepatocytes with stable performance, and quickly and efficiently induces hepatocytes with desired hepatolenticular degeneration phenotypes, while the contamination of matrix cells does not happen.

In some embodiments, the method for preparing the human embryonic stem cell line carrying the ATP7B gene mutation includes the steps of:
culturing an embryo carrying the ATP7B gene mutation in a blastocyst culture medium, mechanically excising an inner cell mass, and culturing the inner cell mass on mouse embryonic fibroblast feeder cells irradiated with gamma rays; and
picking and stably culturing clonal undifferentiated growing cells to generate spreading clones, thereby preparing the hepatolenticular degeneration human embryonic stem cell line.

Specifically, embryos carrying the ATP7B gene mutation are cultured in a blastocyst culture medium for 4 to 6 days, and the inner cell mass is mechanically excised and cultured on mouse embryonic fibroblast feeder cells irradiated with gamma rays. After culturing for 3 to 5 days, the clonal undifferentiated growing cells are picked and further stably cultured. After the stable culture, spreading clones are generated, which are the embryonic stem cell line for hepatolenticular degeneration, namely chHES-391.

It should be noted that the embryos carrying the ATP7B gene mutation in the present application are disease embryos that are within 14 days of fertilization and carry pathogenic mutations without *in vivo* development, and are used to obtain embryonic stem cell lines for hepatolenticular degeneration. The embryonic stem cell lines for hepatolenticular degeneration are *in vitro* induced to differentiate into hepatolenticular degeneration cells with disease phenotypes in the present application.

In some specific embodiments, the method for preparing hepatolenticular degeneration cells further includes:
*in vitro* directedly inducing the human embryonic stem cell line carrying the ATP7B gene mutation to sequentially go through a definitive endoderm induction stage, a liver lineage-directed differentiation stage, and a hepatocyte maturation promotion stage to differentiate into a hepatocyte, thereby forming the hepatolenticular degeneration cell.

Specifically, the embryonic stem cells are initially cultured in a first induction medium for 22 hours to 50 hours, then cultured in a second induction medium for 22 hours to 50 hours, and the second induction medium is replaced every 22 hours to 26 hours during the subsequent culture, so that the embryonic stem cells are induced to differentiate into definitive endoderm cells. The definitive endoderm cells are initially cultured in a third induction medium for 22 hours to 98 hours and then cultured in a fourth induction medium for 118 hours to 170 hours, and the corresponding culture medium is replaced every 22 hours to 26 hours during the culture, so that the definitive endoderm cells are induced to differentiate into liver lineage cells. The liver lineage cells are cultured in a fifth induction medium for 142 hours to 242 hours, and the culture medium is replaced every 22 hours to 26 hours, so that mature hepatocytes are obtained.

In some embodiments, the first induction medium and the second induction medium are sequentially used for induction culture in the definitive endoderm induction stage. Specifically, the first induction medium is a RPMI 1640 medium supplemented with activin A and Wnt3a. In some specific embodiments, a concentration of activin A in the first induction medium is in a range from 5 ng/mL to 300 ng/mL, further 50 ng/mL to 150 ng/mL, furthermore 100 ng/mL. Specifically, the second induction medium is a RPMI 1640 medium supplemented with activin A and fetal bovine serum. A concentration of Wnt3a in the second induction medium is in a range from 20 ng/mL to 100 ng/mL, further 50 ng/mL to 100 ng/mL. A concentration of activin A in the second induction medium is in a range from 10 ng/mL to 200 ng/mL, further 50 ng/mL to 150 ng/mL, furthermore 100 ng/mL. A volume concentration of fetal bovine serum in the second induction medium is in a range from 0.1% to 4%, further 0.2% to 2%.

In some embodiments, the third induction medium and the fourth induction medium are sequentially used for induction culture in the liver lineage-directed differentiation stage. Specifically, the third induction medium is a KO/DMEM medium supplemented with keratinocyte growth factor and fetal bovine serum. In some specific embodiments, a concentration of keratinocyte growth factor is in a range from 15 ng/mL to 100 ng/mL, further can be 15 ng/mL to 70 ng/mL, furthermore can be 30 ng/mL. A volume concentration of fetal bovine serum in the third induction medium is in a range from 1% to 3%, further can be 2%. Specifically, the fourth induction medium is a KO/DMEM medium supplemented with serum substitute, L-glutamine, non-essential amino acids, beta-mercaptoethanol, and dimethyl sulfoxide. In some specific embodiments, a volume concentration of the serum substitute is 20%. A concentration of L-glutamine is in a range from 1 mM to 3 mM, further can be 2 mM to 3 mM. A volume concentration of non-essential amino acids is in a range from 0.1% to 15%, further can be 0.5% to 2%, furthermore can be 1%. A concentration of beta-mercaptoethanol is in a range from 0.1 mM to 0.12 mM. A volume concentration of dimethyl sulfoxide is in a range from 0.2% to 2%, further can be 1% to 2%.

In some embodiments, the fifth induction medium is used for induction culture in the hepatocyte maturation promotion stage. Specifically, the fifth induction medium is an L-15 medium supplemented with hepatocyte growth factor, oncostatin, dexamethasone, L-glutamine, and fetal bovine serum. In some specific embodiments, a concentration of the hepatocyte growth factor is in a range from 10 ng/mL to 300 ng/mL, further can be 10 ng/mL to 200 ng/mL, furthermore can be 10 ng/mL to 100 ng/mL. A concentration of the oncostatin is in a range from 1 ng/mL to 150 ng/mL, further can be 1 ng/mL to 100 ng/mL, furthermore can be 1 ng/mL to 50 ng/mL. A concentration of dexamethasone is in a range from 0.05 µM to 1.2 µM, further can be 0.05 µM to 1.0 µM, furthermore can be 0.5 µM. A concentration of L-glutamine is in a range from 2 mM to 3 mM, further can be 2 mM. A volume concentration of fetal bovine serum in the fifth induction medium is 10%.

A second aspect of the present application provides a hepatolenticular degeneration cell, which is prepared by the above-mentioned preparation method. The hepatolenticular degeneration cell of the present application is a hepatocyte with hepatolenticular degeneration disease phenotypes, which shows typical pathological characteristics of hepatolenticular degeneration disease, such as ATP7B protein functional defects, copper ion metabolism disorders, adverse effects of copper accumulation on hepatocytes, etc., and thus can truly reflect the influence of copper ions on human hepatocytes which is highly correlated with the physiological and pathological conditions of the human body. Therefore, the hepatolenticular degeneration cell provides a new way for hepatolenticular degeneration pathogenic mechanism research, new drug development, and drug hepatotoxicity research, and can improve the efficiency of new drug development for hepatolenticular degeneration disease.

A third aspect of the present application provides a method for evaluating a hepatolenticular degeneration cell, including incubating the hepatolenticular degeneration cell in a culture medium containing copper ions, and detecting at least one of the following in the hepatolenticular degeneration cell before and after incubation: expression levels of ATP7B gene and ATP7B protein, cell vitality, copper ion metabolism capacity, and an oxidative stress response.

Specifically, the copper ions in the culture medium are derived from copper sulfate or copper chloride. The incubation process lasts for 24 hours to 48 hours. The culture medium containing copper ions is replaced every 22 hours to 26 hours. The expression levels of ATP7B gene in the hepatolenticular degeneration cell before and after incubation can be detected by qPCR. The expression levels of ATP7B protein in the hepatolenticular degeneration cell before and after incubation can be detected by the Western blotting method. The copper ion metabolism capacity of the hepatolenticular degeneration cell can be evaluated by detecting the copper ion concentration in the hepatolenticular degeneration cell through atomic absorption spectroscopy. The oxidative stress response in the hepatolenticular degeneration cell can be evaluated by detecting the content of oxidative free radicals in the hepatolenticular degeneration cell through flow cytometry.

In some specific embodiments, when detecting the expression levels of ATP7B gene and ATP7B protein in the hepatolenticular degeneration cell before and after incubation, the copper ion metabolism capacity of the hepatolenticular degeneration cell, and the oxidative stress response in the hepatolenticular degeneration cell, the concentration of copper ions in the culture medium containing copper ions is in a range from 100 µM to 300 µM. When detecting the effect on viability of hepatolenticular degeneration cell by different copper ion concentrations, the concentrations of copper ions in different culture media are in a range from 0 to 1.5 mM. Specifically, the concentration of copper ions in respective culture medium can be 0 mM, 0.5 mM, 1 mM, or 1.5 mM.

In some embodiments, the evaluation method further includes determining the sublocalization of the ATP7B protein in the hepatolenticular degeneration cell. Specifically, the specific distribution of the ATP7B protein in the hepatolenticular degeneration cell can be determined by observing and photographing under a laser confocal microscope.

It is discovered by the present application through the evaluation method that there are significant differences between normal hepatocytes and the hepatolenticular degeneration cells of the present application in terms of the expression levels of ATP7B gene and ATP7B protein, cell viability, copper ion metabolism capacity, oxidative stress response, etc. Specifically, after the incubation with copper ions, the normal hepatocyte shows significantly upregulated ATP7B gene expression level, while the ATP7B gene expression level of the hepatolenticular degeneration hepatocyte does not change significantly. The induced hepatolenticular degeneration hepatocytes hardly express ATP7B protein, and they show a low level of ATP7B protein expression level after incubation with copper ions, while the normal hepatocyte shows significantly increased ATP7B protein expression level after incubation with copper ions. The co-localization ratio of ATP7B protein and Golgi body marker p230 in the hepatolenticular degeneration hepatocyte is significantly lower than the co-localization ratio of ATP7B protein and p230 in the normal hepatocyte. Compared to the normal hepatocyte, the hepatolenticular degeneration hepatocyte shows significantly decreased tolerance to high concentrations of copper ions. The normal hepatocyte and the hepatolenticular degeneration cell of the present application both show significantly different intracellular copper ion contents before and after incubation. The hepatolenticular degeneration hepatocyte shows obvious intracellular copper accumulation after incubation with copper ions, and the hepatolenticular degeneration hepatocyte shows weaker copper ion metabolism capacity than that of the normal hepatocyte. The hepatolenticular degeneration hepatocyte shows higher contents of oxygen free radicals than that of the normal hepatocyte both before and after incubation with copper ions, which indicates that the hepatolenticular degeneration hepatocyte is more affected by the oxidative stress response caused by copper accumulation.

A third aspect of the present application provides use of the hepatolenticular degeneration cell in the manufacture of a medicament for treating hepatolenticular degeneration.

It can be understood that the present application creatively *in vitro* induces the human embryonic stem cell carrying specific ATP7B gene mutations to differentiate into hepatocytes to obtain hepatolenticular degeneration cells, which can truly reflect the influence of copper ions on phenotypes of hepatolenticular degeneration cells, including expression levels of ATP7B gene and ATP7B protein, copper ion metabolism capacity, oxidative stress response, cell viability, etc., and is highly correlated with the physiological and pathological conditions of the human body. Therefore, the present application provides a new way for hepatolenticular degeneration pathogenic mechanism research, new drug development, and drug hepatotoxicity research, and can improve the efficiency of new drug development for hepatolenticular degeneration disease.

The embodiments of the present application are described in detail below with reference to Examples.

Reagents and equipment used in Examples are as follows.

*ch*HES-391 (embryonic stem cells): A human embryonic stem cell line with hepatolenticular degeneration, established by the National Engineering Research Center for Human Stem Cells from discarded embryos carrying ATP7B gene pathogenic mutations obtained through preimplantation genetic diagnosis. The ATP7B gene mutations include the maternal mutation c.1881_1882insT and the paternal mutation c.2333G>T.

*ch*HES-90 (embryonic stem cells): A normal human embryonic stem cell line previously established by the National Engineering Research Center for Human Stem Cells.

The research on embryo establishment follows the GMP process and the corresponding ethical principles of human tissue research. With full informed consents of patients, abnormal or normal embryos voluntarily donated by patients undergone assisted reproductive treatment were used to isolate diseased human embryonic stem cell lines or normal human embryonic stem cell lines.

### Cell culture reagents:

Human activin A (R&D, 338-AC-050), indicating that it was purchased from R&D Systems, with a catalog number: 338-AC-050. The same applies below. Wnt3a (R&D, 1324-WN-010), fetal bovine serum (Gibco, 10099-141), keratinocyte growth factor (KGF) (R&D, 251-KG-050), serum substitute (Life, N10828-028), L-glutamine (Gibco, 25030-081), non-essential amino acid (Gibco, 1140-050), beta-mercaptoethanol (Gibco, 21385-023), dimethyl sulfoxide (DMSO) (Sigma, D2660), hepatocyte growth factor (HGF) (R&D, 294-HG-025), oncostatin (OSM) (R&D, 295-OM-050), dexamethasone (Calbiochem, 265005), mTESR (Stem cell, 05850), RPMI 1640 (Life, 11875-085), KO/DMEM (Gibco, A1286101), L-15 (Gibco, 21083-027), Matrigel matrix (BD, 354234).

4. Other reagents: copper sulfate (Sigma Aldrich, 451657), copper chloride (Sigma Aldrich, 203149), CCK-8 (Sangon Biotech, E606335), CM-H2DCFDA (Invitrogen, C6827).

### Example 1 Establishment of an embryonic stem cell line for hepatolenticular degeneration

Experimental objects: Abandoned embryos carrying ATP7B gene mutations obtained through preimplantation genetic diagnosis were taken as experimental objects. The ATP7B gene mutations included the maternal mutation c.1881_1882insT and the paternal mutation c.2333G>T. The experimental samples were from Xiangya Hospital. The research in the example was discussed and approved by the Ethics Committee of CITIC Xiangya Reproductive and Genetic Specialty Hospital, with full informed consents of patients. The personal information of donors was strictly confidential. The human embryonic stem cell line carrying the above-mentioned ATP7B gene mutations was established from embryonic stem cells derived from the abnormal embryos according to the following specific process.

Embryos carrying the above-mentioned ATP7B gene mutations were cultured in a blastocyst culture medium for 4 days to 6 days, and the inner cell mass was mechanically excised and cultured on mouse embryonic fibroblast feeder cells (MEF) irradiated with gamma rays. After culturing for 3 days to 5 days, the clonal undifferentiated growing cells were picked and further stably cultured. After the stable culture, spreading clones were generated, which were the embryonic stem cell line carrying the above-mentioned ATP7B gene mutations, namely *ch*HES-391.

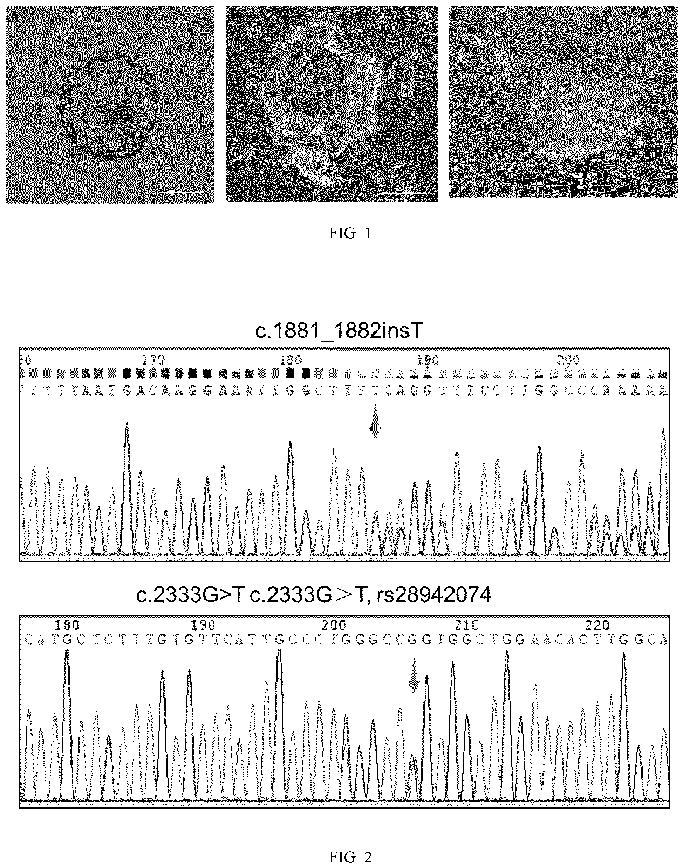

FIG. 1 shows the embryo carrying the above-mentioned ATP7B gene mutations incubated *in vitro* for six days. After culturing for 3 days to 5 days, small clones were generated, the *ch*HES-391 cell line obtained after stably culturing on the MEF cells showed clonal growing state, with compact cells and a large nuclear-cytoplasmic ratio, which can be used for the next experiment.

The ATP7B gene was sequenced according to the specific process as below. The genomic DNA was extracted using a DNA mini kit, and sequenced via the Next-Generation Sequencing (NGS) to analyze the ATP7B gene mutation. The primers for PCR amplification reaction included a forward primer (5'-ATATTGAGCGGTTACAAAGCACT-3') and a reverse primer (5'-TGCCCCAAGGTCTCAGAATTA-3').

As shown in FIG. 2, *ch*HES-391 carried ATP7B gene mutations, which are compound heterozygous mutations. The maternally inherited insertion mutation c.1881_1882insT is a new pathogenic mutation that has not been previously reported. The paternally inherited missense mutation c.2333G > T (p.R778L) is the most common pathogenic mutation in Asians.

### Example 2 Directed induction and differentiation of the chHES-391 cell into hepatocytes

Induction and differentiation of *ch*HES-391 cells: When the *ch*HES-391 cells were in the best growth state on the feeder cells, the cells were artificially picked and subcultured in culture dishes pre-coated with Matrigel. After cultured in the mTesR1 culture system for 2 days to 4 days, the cells were cultured in induction mediums. The specific induction process was as follows:

In a first stage (3 days to 6 days), *ch*HES-391 cells were induced into definitive endoderm cells. On days 1 to 2, the induction medium was a RPMI 1640 minimum medium supplemented with human activin A and Wnt3a, in which the concentration of human activin A was 5 ng/mL to 300 ng/mL, and the concentration of Wnt3a was 20 ng/mL to 100 ng/mL. On days 3 to 4, the induction medium was a RPMI 1640 minimum medium supplemented with human activin A and fetal bovine serum, in which the final concentration of human activin A was 10 ng/mL to 200 ng/mL, and the final concentration of fetal bovine serum was 0.1% to 4%. On days 5 to 6, the induction medium was a RPMI 1640 minimum medium supplemented with human activin A and fetal bovine serum, in which the final concentration of human activin A was 10 ng/mL to 200 ng/mL, and the final concentration of fetal bovine serum was 0.1% to 4%. In the first few days of induction, many cells were dead. In order to avoid the dead cells affecting the induction process, the culture medium was removed as clean as possible, and the cells were washed one or two times with RPMI 1640 before replacing the culture medium.

In a second stage (6 days to 12 days), the definitive endoderm cells were directedly differentiated into liver lineage cells. In the first 1 day to 4 days of the second stage, the culture medium used was a KO/DMEM minimum medium supplemented with keratinocyte growth factor of 15 ng/mL to100 ng/mL and fetal bovine serum with a final concentration of 1% to 3%. During the culture in the second stage, the number of cells was significantly increased over the previous few days, and thus the added amount of the culture medium should be appropriate for the number of cells to ensure the normal growth of the cells. In the next 5 days to 12 days, the culture medium was a KO/DMEM minimum medium supplemented with serum substitute (SR), L-glutamine, non-essential amino acids, beta-mercaptoethanol, and dimethyl sulfoxide, and the culture medium was replaced every 24 hours, in which a final concentration of serum substitute was 20%, a final concentration of L-glutamine was 1 mM to 3 mM, a final concentration of non-essential amino acid was 0.1% to 15%, a final concentration of beta-mercaptoethanol was 0.1 mM to 0.12 mM, and a final concentration of dimethyl sulfoxide was 0.2% to 2%.

A third stage (6 to 10 days) is a hepatocyte maturation promotion stage. The culture medium is an L-15 minimum medium supplemented with hepatocyte growth factor, oncostatin, dexamethasone, L-glutamine, and fetal bovine serum. The culture medium was replaced every 24 hours. The final concentration of fetal bovine serum was 10%. A final concentration of hepatocyte growth factor was in a range from 10 ng/mL to 300 ng/mL. A final concentration of oncostatin was in a range from 5 ng/mL to 150 ng/mL. A final concentration of dexamethasone was in a range from 0.05 µM to 1.2 µM. A final concentration of L-glutamine was in a range from 2 mM to 3 mM.

The entire induction process lasted 15 to 28 days.

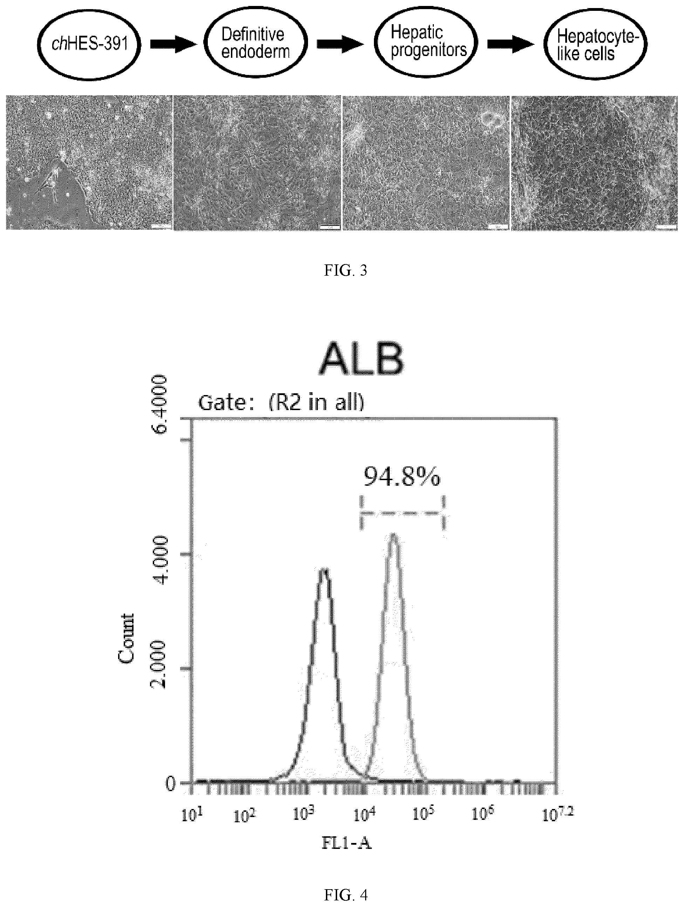

As shown in FIG. 3, *ch*HES-391 cells have a high nuclear-cytoplasmic ratio, are closely arranged, and grow in a clonal manner. After the induction begins, the morphology of the cells begins to change. After 3 days of induction, the cells develop into the definitive endoderm, and the cells become flat, and oval shaped. After entering the second induction stage, the cells begin hepatocyte specialization, and the cells gradually develop a well-defined irregular polygonal epithelial cell-like morphology. In the third stage, the cells develop into mature hepatocytes, in which the cells have increased sizes, are arranged in irregular polygons, with distinct round cell nucleus and rich cytoplasm granules, and cells with two or more nuclei are observed.

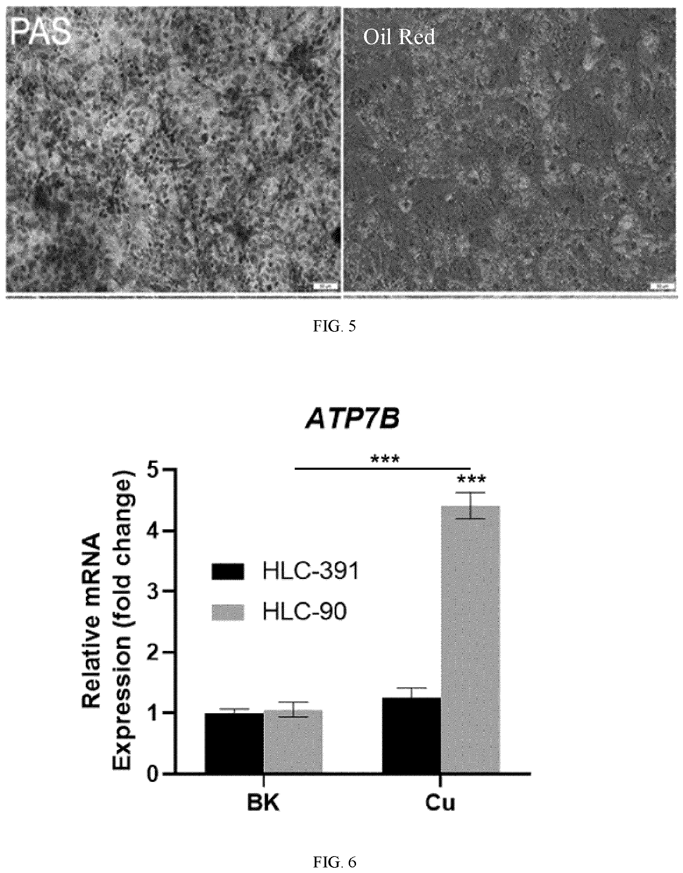

The induced hepatolenticular degeneration HLC-391 cells were identified, and the results are shown in FIG. 4. As shown in FIG. 4, the expression level of mature hepatocyte marker ALB in the induced hepatolenticular degeneration HLC-391 cells was identified by flow cytometry, and the positive rate was up to 94.8%, which shows that the present induction system achieves a high hepatocyte differentiation efficiency, and obtains hepatocytes with high purity. The HLC-391 cells were further detected by PAS glycogen staining and Oil Red-O staining experiments to evaluate the glycogen synthesis and storage function and the lipid synthesis and storage function. The staining results are shown in FIG. 5. As shown in FIG. 5, after HLC-391 cells were stained by the PAS method for glycogen, a large number of purple-red particles were observed in the cytoplasm of most cells, indicating that the induced HLC-391 cell has a strong glycogen synthesis and storage function. After HLC-391 cells were stained with Oil Red-O, red lipid droplet particles were observed in most cells under an inverted phase contrast microscope, indicating that HLC-391 cell has the lipid synthesis and storage function.

### Example 3 Evaluation of an in vitro disease model of hepatolenticular degeneration

3.1 Detection of ATP7B gene expression: Cells on the last day of induction were collected, and ATP7B gene expression levels in the hepatocytes before and after incubation with 100 µM to 300 µM of copper sulfate or copper chloride were detected by qPCR method, with GAPDH gene as the internal reference. Primers were used for validation. The qPCR method was performed as follows: 95°C for 5 minutes; 45 cycles (94°C for 40 seconds, 54°C to 58°C for 10 seconds, 72°C for 10 seconds); and 72°C extension for 5 minutes.

As shown in FIG. 6, the induced hepatolenticular degeneration hepatocytes and the normal hepatocytes both can normally express the ATP7B gene at the transcriptional level. However, after incubation with copper ions, the expression level of the ATP7B gene in the normal hepatocytes was significantly upregulated, while the expression level of the ATP7B gene in the hepatolenticular degeneration hepatocytes was not upregulated significantly.

3.2 Detection of ATP7B protein expression: Cells on the last day of induction were collected, and ATP7B protein expression levels in the hepatocytes before and after incubation with 100 µM to 300 µM of copper sulfate or copper chloride were detected by Western blotting method, with GAPDH protein as the internal reference. The induced cells were digested and collected in a 1.5 mL EP tube. An appropriate amount of protein lysis buffer was added depending on the cell solution volume (adding 200 µL RIPA lysis buffer for 1×10⁶ cells). The cell solution was pipetted up and down to mix, and the cells were lysed in an ice bath with an ultrasonic cell disruptor for 15 minutes, then centrifuged at 13000 ×g and 4°C for 10 minutes. The supernatant was transferred to a new EP tube, and the protein concentration was detected using a BCA protein quantification kit. Polyacrylamide gel for SDS-PAGE was prepared, including preparing an 8% separating gel and a stacking gel. After the stacking gel was solidified, the comb was carefully pulled out. After a 40 µg sample and a 5 µL protein marker were loaded, the electrophoresis started at a 110 V constant voltage. After the Loading Buffer ran out of the separating gel, the electrophoresis stopped. A wet transfer apparatus was assembled according to a sequence of a positive electrode, sponge, filter paper, nitrocellulose membrane, the gel, filter paper, a sponge, and a negative electrode, during which bubbles should be prevented from generating between layers. Proteins were transferred to the nitrocellulose membrane at 220 mA constant current for 2 hours. After that, the nitrocellulose membrane was taken out, marked, and cut to collect the proteins. The membrane containing the proteins was blocked with 5% skim milk at room temperature for 1 hour. A primary antibody was diluted with a 5% skim milk according to the following dilution ratios: 1:5000 for ATP7B, and 1:10000 for GAPDH. The membrane was transferred to the primary antibody dilution solution and incubated at 4 °C overnight. Then, the membrane was washed three times with a TBST solution to remove the unbound primary antibody, 10 minutes each washing. A secondary antibody was diluted with a TBST solution at 1:5000. The membrane was transferred to the secondary antibody dilution solution and incubated at room temperature for 1 hour. Then, the membrane was washed three times with a TBST solution to remove the unbound secondary antibody, 10 minutes each washing. In a dark room, solution A and solution B in a chemiluminescent reagent kit were mixed at a ratio of 1:1. Next, the membrane was transferred to the chemiluminescent reagent solution and soaked for 10 seconds to 10 minutes. Subsequently, the membrane was imaged by using a chemiluminescence imaging system to analyze the expression level of the protein.

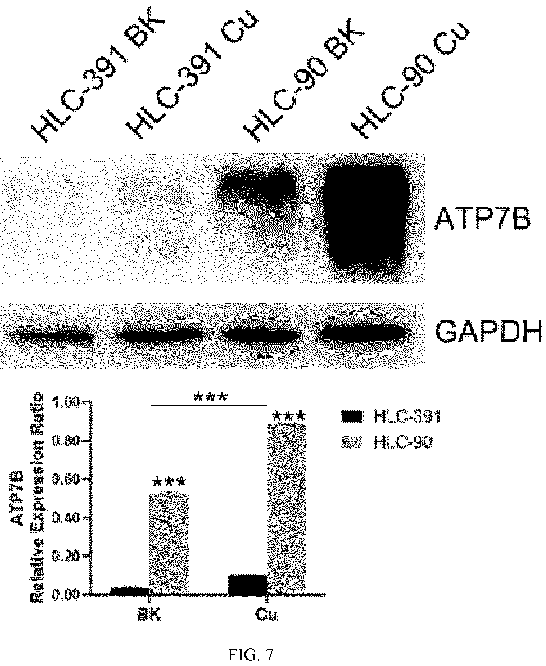

As shown in FIG. 7, the induced hepatolenticular degeneration hepatocyte barely expresses the ATP7B protein. After incubation with copper ions, the induced hepatolenticular degeneration hepatocyte still expresses a low level of the ATP7B protein, while the expression level of the ATP7B protein in the normal hepatocyte is significantly increased.

3.3 Intracellular sublocalization of the ATP7B protein: On the last day of induction, the culture medium was discarded, the cells were washed with a DPBS solution once to remove the residual culture medium and cell debris. The cells were fixed with 4% paraformaldehyde at room temperature for 15 minutes. The paraformaldehyde was discarded, and the cells were washed with a DPBS solution twice to remove the residual paraformaldehyde. Then, the cells were permeabilized with 0.2% Triton X-100 at room temperature for 10 minutes, followed by discarding Triton X-100, and washing with DPBS twice. The cells were blocked with sheep serum working solution for 1 hour, and the blocking solution was discarded. A diluted primary antibody solution was directly added to the cells (the dilution ratio was 1:250 for ATP7B, and 1:250 for p230), incubated at 4°C overnight, followed by discarding the primary antibody, and washing as clean as possible with DPBS three times. Next, a diluted secondary antibody Alexa Fluor 488/594 (1:1000) was added to the cells, incubated at room temperature for 1 hour in the dark, followed by discarding the secondary antibody, and washing with DPBS three times. The cells were incubated with DAPI (1:1000) for 3 minutes at room temperature in the dark. The DAPI was discarded, and the cells were washed three times with DPBS and then immersed in DPBS for observation and photography under a laser confocal microscope.

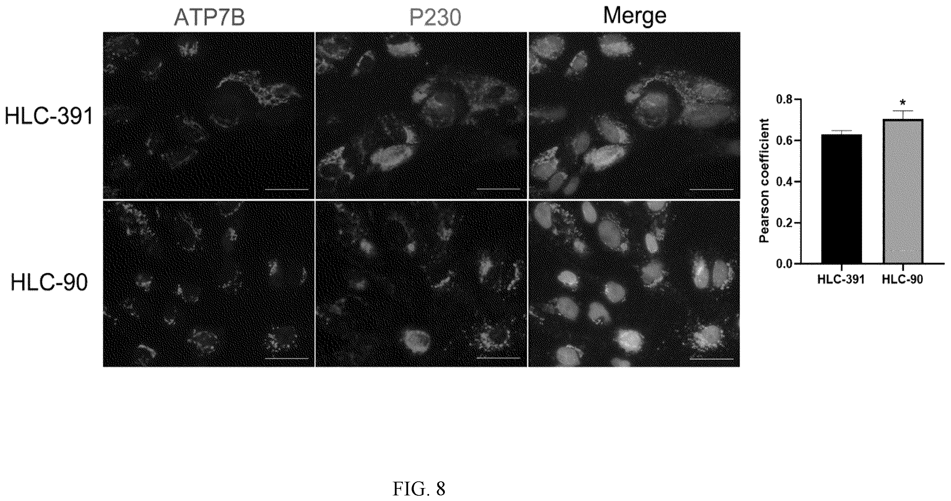

As shown in FIG. 8, the co-localization ratio of ATP7B protein and Golgi marker p230 in the hepatolenticular degeneration hepatocytes is 62%, while the co-localization ratio of ATP7B protein and p230 in the normal hepatocytes is 70%, with a significant difference (P<0.05).

3.4 Effect of copper ion accumulation on cell viability: The induced cells were digested and evenly inoculated in a 96-well plate, with about 3×10⁴ cells per well, and edge wells were filled with sterile PBS. After the cells were adhered to the wall, a copper sulfate or copper chloride solution in different concentrations (0 mM, 0.5 mM, 1 mM, and 1.5 mM) were added, and 6 replicate wells were set in each group. The wells without cells were set as blank wells. After incubation at 37°C for 48 hours (fresh culture medium was replaced every 22 hours to 26 hours), the original culture medium was discarded, and the cells were washed three times with DPBS. Fresh culture medium (100 µL per well) was added, and a 10 µL CCK-8 reaction solution was added to each well, followed by incubating at 37 °C in the dark for 4 hours. After the incubation, the absorbance at 450 nm was detected using a microplate reader.

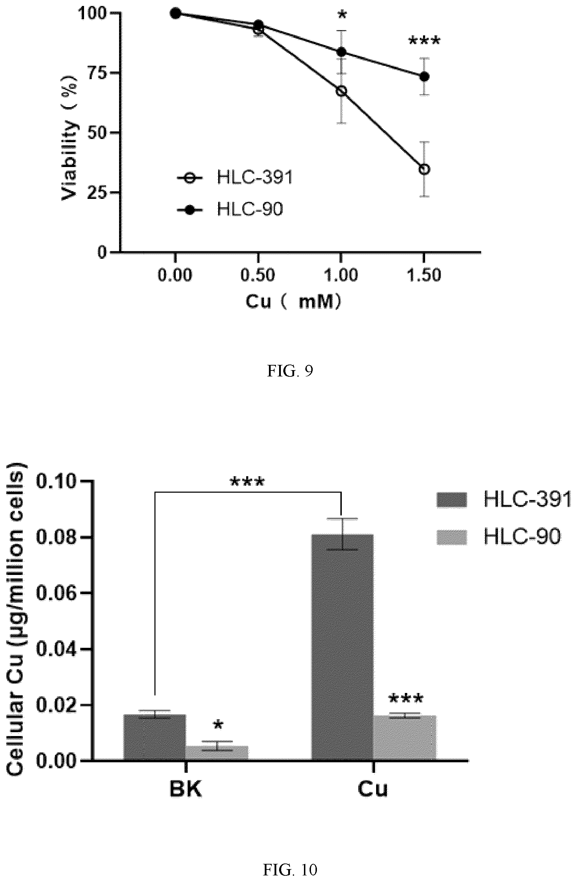

As shown in FIG. 9, in the absence of incubation with copper ions, both groups show 100% cell viability. After incubation with 0.5 mM copper ions for 48 hours, the cell viability of the hepatolenticular degeneration hepatocytes is 93.25%, while the cell viability of the normal hepatocytes is 95.33%. After incubation with 1 mM copper ions for 48 hours, the cell viability of the hepatolenticular degeneration hepatocytes decrease to 67.49%, while the cell viability of the normal hepatocytes is 83.78%. After incubation with 1.5 mM copper ions for 48 hours, the cell viability of the hepatolenticular degeneration hepatocytes decrease to 34.8%, while the cell viability of the normal hepatocytes is 73.53%. The results show that the hepatolenticular degeneration hepatocytes have significantly lower tolerance to high concentrations of copper ions than that of normal hepatocytes.

3.5 Intracellular copper ion metabolism capacity: The induced hepatolenticular degeneration hepatocytes were incubated in a 0.1 mM copper sulfate or copper chloride solution for 48 hours, and normal hepatocytes were incubated in the same solution. The culture medium was replaced every 22 to 26 hours. After the treatment, the cells were washed three times with DPBS, an appropriate amount of trypsin was added to digest the cells, and the digested cells were collected in a 15 mL centrifuge tube. The cells were counted to adjust the density of cells to about 1×10⁶ /mL. After the cells were lysed by ultrasonication, the lysis solution was acidified with 3% nitric acid solution, followed by centrifuged at 12000 ×g for 5 minutes. The supernatant was collected, and the copper ion content in the supernatant was measured by using an atomic absorption spectrometer.

As shown in FIG. 10, before the incubation, the intracellular copper ion concentration of the hepatolenticular degeneration hepatocytes is 0.0167 µg/million cells, while the intracellular copper ion concentration of the normal hepatocytes is 0.0054 µg/million cells. After incubation with copper ions for 48 hours, the intracellular copper ion concentration of the hepatolenticular degeneration hepatocytes is 0.0812 µg/million cells, while the intracellular copper ion concentration of the normal hepatocytes is 0.0163 µg/million cells. Before and after the incubation, the cells in both groups show significantly different intracellular copper ion contents, meanwhile the hepatolenticular degeneration hepatocytes show obvious intracellular copper ion accumulation after incubation with copper ions, which indicates that the copper ion metabolism ability of the hepatolenticular degeneration hepatocytes is weaker than that of the normal hepatocytes.

3.6 Effect of copper ion accumulation on intracellular oxidative stress response: The induced hepatolenticular degeneration hepatocytes were digested with trypsin, and the cells were counted to adjust the density of cells to 1×10⁶ /mL. The cells were resuspended with a 200 µL preheated working solution and incubated at 37°C in dark for 30 minutes. The cell suspension was centrifuged at 1500 rpm to remove the supernatant containing CM-H2DCFDA. The cells were gently washed twice with preheated DPBS or serum-free culture medium and then analyzed by using a BD Accuri C6 instrument.

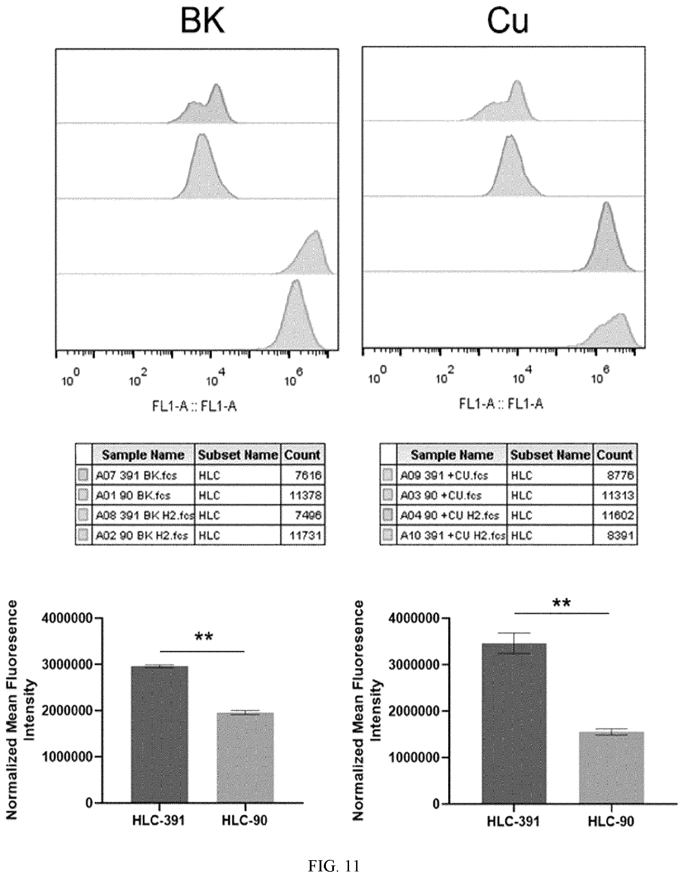

As shown in FIG. 11, before and after incubation with copper ions, the hepatolenticular degeneration hepatocytes show higher oxygen free radical content that of the normal hepatocytes, which indicates that the hepatolenticular degeneration hepatocytes are more affected by the oxidative stress response caused by copper ion accumulation.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A method for preparing a hepatolenticular degeneration cell, comprising *in vitro* directedly inducing a human embryonic stem cell line carrying an ATP7B gene mutation to differentiate into a hepatocyte thereby forming the hepatolenticular degeneration cell.

2. The method according to claim 1, wherein the ATP7B gene mutation satisfies at least one of following characteristics:
(1) the ATP7B gene mutation comprises a c.1881_1882insT homozygous mutation;
(2) the ATP7B gene mutation comprises a c.2333G>T homozygous mutation; and
(3) the ATP7B gene mutations comprise c.2333G>T and c.1881_1882insT compound heterozygous mutations.

3. The method according to claim 1 or 2, wherein the method further comprises:
*in vitro* directedly inducing the human embryonic stem cell line carrying the ATP7B gene mutation to sequentially go through a definitive endoderm induction stage, a liver lineage-directed differentiation stage, and a hepatocyte maturation promotion stage to differentiate into a hepatocyte, thereby forming the hepatolenticular degeneration cell.

4. The method according to claim 3, wherein a first induction medium and a second induction medium are sequentially used in the definitive endoderm induction stage,
a third induction medium and a fourth induction medium are sequentially used for induction culture in the liver lineage-directed differentiation stage, and
a fifth induction medium is used in the hepatocyte maturation promotion stage,
wherein the first induction medium is a RPMI 1640 medium supplemented with activin A and Wnt3a;
the second induction medium is a RPMI 1640 medium supplemented with activin A and fetal bovine serum;
the third induction medium is a KO/DMEM medium supplemented with keratinocyte growth factor and fetal bovine serum;
the fourth induction medium is a KO/DMEM medium supplemented with a serum substitute, L-glutamine, a non-essential amino acid, beta-mercaptoethanol, and dimethyl sulfoxide; and the fifth induction medium is an L-15 medium supplemented with hepatocyte growth factor, oncostatin, dexamethasone, L-glutamine, and fetal bovine serum.

5. The method according to any one of claims 1 to 4, wherein the method for preparing the human embryonic stem cell line carrying the ATP7B gene mutation comprises the steps of:
culturing an embryo carrying the ATP7B gene mutation in a blastocyst culture medium,
mechanically excising an inner cell mass, and culturing the inner cell mass on mouse embryonic fibroblast feeder cells irradiated with gamma rays; and
picking and stably culturing clonal undifferentiated growing cells to generate spreading clones, thereby preparing the embryonic stem cell line for hepatolenticular degeneration.

6. A hepatolenticular degeneration cell, prepared by the method according to any one of claims 1 to 5.

7. A method for evaluating a hepatolenticular degeneration cell, comprising incubating the hepatolenticular degeneration cell according to claim 6 in a culture medium containing copper ions, and evaluating effects of copper ions on the hepatolenticular degeneration cell before and after incubation.

8. The method according to claim 7, wherein evaluating the effects of copper ions on the hepatolenticular degeneration cell before and after incubation specifically comprises:
detecting at least one of the following in the hepatolenticular degeneration cell before and after incubation: expression levels of ATP7B gene and ATP7B protein, sublocalization of ATP7B protein, cell vitality, copper ion metabolism capacity, and an oxidative stress response.

9. The method according to claim 8, wherein when at least one of the expression levels of ATP7B gene and ATP7B protein, the copper ion metabolism capacity, and the oxidative stress response in the hepatolenticular degeneration cell before and after incubation is detected, a concentration of copper ions is in a range from 100 µM to 300 µM; and/or detecting the cell vitality of the hepatolenticular degeneration cell before and after incubation specifically comprises detecting the cell viability of the hepatolenticular degeneration cell in different culture media containing different concentrations of copper ions before and after incubation, wherein the concentrations of copper ions in the different culture media are in a range from 0 mM to 1.5 mM.

10. Use of the hepatolenticular degeneration cell according to claim 6 in the manufacture of a medicament for treating a hepatolenticular degeneration disease.
